# EUROPÄISCHE PATENTSCHRIFT

(11) **EP 1 073 653 B1**
(45) Veröffentlichungstag und Bekanntmachung des Hinweises auf die Patenterteilung: **28.08.2002**
(21) Anmeldenummer: 99915743.1
(22) Anmeldetag: 07.04.1999
(51) Int. Cl.: C07D 417/04, C07D 413/04, C07D 417/14, A61K 31/425

(54) **NEUE 2-HETEROCYCLISCH SUBSTITUIERTE DIHYDROPYRIMIDINE**
NOVEL 2-HETEROCYCLICALLY SUBSTITUTED DIHYDROPYRIMIDINES
NOUVELLES DIHYDROPYRIMIDINES A SUBSTITUTION 2-HETEROCYCLIQUE

(30) Priorität: 18.04.1998 DE 19817262
(43) Veröffentlichungstag der Anmeldung: 07.02.2001
(73) Patentinhaber: BAYER AG, 51368 Leverkusen (DE)
(72) Erfinder: GOLDMANN, Siegfried, D-42327 Wuppertal (DE); STOLTEFUSS, Jürgen, D-42781 Haan (DE); PAESSENS, Arnold, D-42781 Haan (DE); GRAEF, Erwin, D-42553 Velbert (DE); LOTTMANN, Stefan, D-42115 Wuppertal (DE)
(86) Internationale Anmeldenummer: EP9902345
(87) Internationale Veröffentlichungsnummer: WO99054329

(56) Entgegenhaltungen:
- EP-A- 0 103 796

## Beschreibung

Die vorliegende Erfindung betrifft neue 2-heterocyclisch substituierte Dihydropyrimidine, Verfahren zu ihrer Herstellung und ihre Verwendung als Arzneimittel, insbesondere als Arzneimittel zur Behandlung und Prophylaxe von Hepatitis B.

Aus der Publikation EP 103 796 A2 sind bereits Dihydropyrimidine mit einer kreislaufbeeinflussenden Wirkung bekannt.

Die vorliegende Erfindung betrifft jetzt neue 2-heterocyclisch substituierte Dihydropyrimidine der allgemeinen Formel (I) 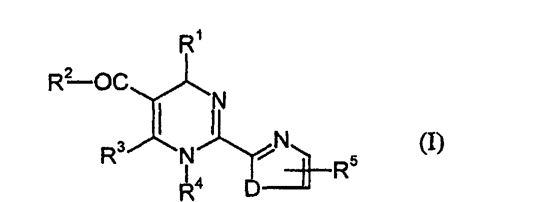 bzw. deren isomere Form (Ia) 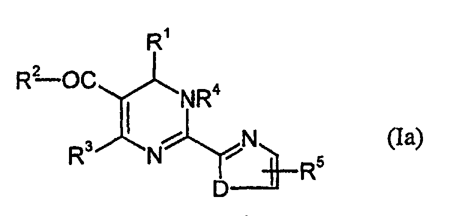 in welchen
- R¹: für Phenyl, Furyl, Thienyl, Pyridyl, Cycloalkyl mit 3 bis 6 Kohlenstoffatomen oder für einen Rest der Formel 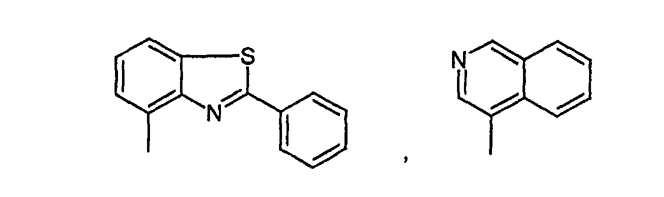 oder 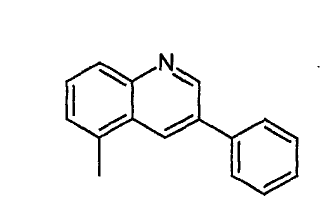 steht,
wobei die oben aufgeführten Ringsysteme gegebenenfalls einfach oder mehrfach, gleich oder verschieden durch Substituenten, ausgewählt aus der Gruppe Halogen, Trifluormethyl, Nitro, Cyano, Trifluormethoxy, Carboxyl, Hydroxyl, (C₁-C₆)-Alkoxy, (C₁-C₆)-Alkoxycarbonyl und (C₁-C₆)-Alkyl substituiert sind, das seinerseits durch Aryl mit 6 bis 10 Kohlenstoffatomen oder Halogen substituiert sein kann,
und/oder die aufgeführten Ringsysteme gegebenenfalls durch Gruppen der Formel -S-R⁶, NR⁷R⁸, CO-NR⁹R¹⁰, SO₂-CF₃ und -A-CH₂-R¹¹ substituiert sind,
worin
R⁶ Phenyl bedeutet, das gegebenenfalls durch Halogen substituiert ist,
R⁷, R⁸, R⁹ und R¹⁰ gleich oder verschieden sind und Wasserstoff, Phenyl, Hydroxy-substituiertes Phenyl, Hydroxy, (C₁-C₆)-Acyl oder (C₁-C₆)-Alkyl bedeuten, das seinerseits durch Hydroxy, (C₁-C₆)-Alkoxycarbonyl, Phenyl oder Hydroxy substituiertes Phenyl substituiert sein kann,
A einen Rest O, S, SO oder SO₂ bedeutet,
R¹¹ Phenyl bedeutet, das gegebenenfalls ein- bis mehrfach, gleich oder verschieden durch Substituenten, ausgewählt aus der Gruppe Halogen, Nitro, Trifluormethyl, (C₁-C₆)-Alkyl und (C₁-C₆)-Alkoxy substituiert ist,
- R²: für einen Rest der Formel -OR¹² oder -NR¹³R¹⁴ steht,
worin
R¹² Wasserstoff, (C₁-C₆)-Alkoxycarbonyl oder einen geradkettigen, verzweigten oder cyclischen, gesättigten oder ungesättigten (C₁-C₈)-Kohlenwasserstoffrest bedeutet, der gegebenenfalls eine oder zwei gleiche oder verschiedene Heterokettenglieder aus der Gruppe O, CO, NH, -N-(C₁-C₄)-Alkyl,-N-((C₁-C₄)-Alkyl)₂, S und SO₂ enthält und der gegebenenfalls substituiert ist durch Halogen, Nitro, Cyano, Hydroxy, Aryl mit 6 bis 10 Kohlenstoffatomen oder Aralkyl mit 6 bis 10 Kohlenstoffatomen, Heteroaryl oder einer Gruppe der Formel -NR¹⁵R¹⁶,
worin
R¹⁵ und R¹⁶ gleich oder verschieden sind und Wasserstoff, Benzyl oder (C₁-C₆)-Alkyl bedeuten,
R¹³ und R¹⁴ gleich oder verschieden sind und Wasserstoff, (C₁-C₆)-Alkyl oder Cycloalkyl mit 3 bis 6 Kohlenstoffatomen bedeuten,
- R³: für Wasserstoff,Amino oder
für einen Rest der Formel 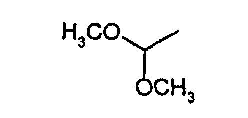 steht, oder
für Formyl, Cyano oder Trifluormethyl steht, oder
für einen geradkettigen, verzweigten oder cyclischen, gesättigten oder ungesättigten Kohlenwasserstoffrest mit bis zu 8 Kohlenstoffatomen steht, der gegebenenfalls ein- oder mehrfach, gleich oder verschieden durch Aryloxy mit 6 bis 10 Kohlenstoffatomen, Azido, Cyano, Hydroxy, Carboxyl, (C₁-C₆)-Alkoxycarbonyl, einen 5- bis 7-gliedrigen heterocyclischen Ring, (C₁-C₆)-Alkylthio, (C₁-C₆)-Alkoxy substituiert ist, das seinerseits durch Azido oder Amino substituiert sein kann,
und/oder durch Triazolyl substituiert ist, das seinerseits bis zu 3-fach durch (C₁-C₆)-Alkoxycarbonyl substituiert sein kann,
und/oder durch Gruppen der Formel -OSO₂-CH₃ oder (CO)ₐ-NR¹⁷R¹⁸ substiutiert sein kann,
worin
a eine Zahl 0 oder 1 bedeutet,
R¹⁷ und R¹⁸ gleich oder verschieden sind und Wasserstoff oder Aryl, Aralkyl mit 6 bis 10 Kohlenstoffatomen bedeuten,
oder (C₁-C₆)-Alkyl bedeuten, das gegebenenfalls durch (C₁-C₆)-Alkoxycarbonyl, Hydroxyl, Phenyl oder Benzyl substituiert ist, wobei Phenyl oder Benzyl gegebenenfalls ein- oder mehrfach, gleich oder verschieden durch Hydroxy, Carboxyl, (C₁-C₆)-Alkyl oder (C₁-C₆)-Alkoxy substituiert sind,
oder (C₁-C₆)-Alkyl gegebenenfalls durch Gruppen der Formel NH-CO-CH₃ oder NH-CO-CF₃ substituiert ist,
- R⁴: für Wasserstoff, (C₁-C₄)-Alkyl, Acetyl oder Benzoyl steht,
- D: für ein Sauerstoff- oder Schwefelatom steht,
- R⁵: für Wasserstoff, Halogen oder für geradkettiges oder verzweigtes Alkyl mit bis zu 6 Kohlenstoffatomen steht,
und deren Salze.

Bevorzugt werden erfindungsgemäße Verbindungen der allgemeinen Formeln (I) bzw. (Ia)
in welchen
- R¹: für Phenyl, Furyl, Thienyl, Pyridyl, Cyclopentyl oder Cyclohexyl steht, wobei die oben aufgeführten Ringsysteme gegebenenfalls ein- oder 2-fach gleich oder verschieden durch Substituenten, ausgewählt aus der Gruppe Halogen, Trifluormethyl, Nitro, SO₂-CF₃, Methyl, Cyano, Trifluormethoxy, Carboxyl, Methoxycarbonyl oder Resten der Formel -CO-NH-CH₂-C(CH₃)₃, -CO-NH(CH₂)₂OH, -CO-NH-CH₂-C₆H₅, -CO-NH-C₆H₅, -CO-NH-(pOH)-C₆H₄, -O-CH₂-C₆H₅ oder -S-pCl-C₆H₄ substituiert sind,
- R²: für einen Rest der Formel -OR¹² oder -NR¹³R¹⁴ steht,
worin
R¹² Wasserstoff, (C₁-C₄)-Alkenyl oder (C₁-C₄)-Alkyl bedeutet, das gegebenenfalls durch Pyridyl, Cyano, Phenoxy, Benzyl oder durch einen Rest der Formel -NR¹⁵R¹⁶ substituiert ist,
worin
R¹⁵ und R¹⁶ gleich oder verschieden sind und Wasserstoff, Methyl oder Ethyl bedeuten,
R¹³ und R¹⁴ gleich oder verschieden sind und Wasserstoff, Methyl, Ethyl oder Cyclopropyl bedeuten,
- R³: für Wasserstoff oder einen Rest der Formel 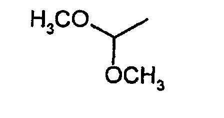 steht,
oder
für Formyl, Cyano, Trifluormethyl oder Cyclopropyl steht, oder
für (C₁-C₄)-Alkyl steht, das gegebenenfalls durch Reste der Formel -SO₂CH₃, -NH-CO-CH₃, -NH-CO-CF₃, Fluor, Chlor, (C₁-C₃)-Alkoxycarbonyl oder Hydroxy, substituiert ist,
und/oder Alkyl gegebenenfalls durch Gruppen der Formel -OSO₂-CH₃ oder (CO)ₐ-NR¹⁷R¹⁸ substituiert ist,
worin
a eine Zahl 0 oder 1 bedeutet,
R¹⁷ und R¹⁸ gleich oder verschieden sind und Wasserstoff, Phenyl oder Benzyl bedeuten, oder
C₁-C₄-Alkyl bedeuten, das gegebenenfalls durch (C₁-C₄)-Alkoxycarbonyl, Hydroxyl, Phenyl oder Benzyl substituiert ist, wobei Phenyl oder Benzyl gegebenenfalls ein- oder mehrfach gleich oder verschieden durch Hydroxy, Carboxy, (C₁-C₄)-Alkyl oder (C₁-C₄)-Alkoxy substituiert sind,
und/oder (C₁-C₄)-Alkyl gegebenenfalls durch Reste der Formel -NH-CO-CH₃ oder -NH-CO-CF₃ substituiert ist,
oder
- R⁴: für Wasserstoff, Methyl, Ethyl oder Acetyl steht,
- D: für ein Sauerstoff- oder Schwefelatom steht,
- R⁵: für Wasserstoff, Halogen oder für (C₁-C₄)-Alkyl steht,
und deren Salze.

Besonders bevorzugt werden erfindungsgemäße Verbindungen der allgemeinen Formeln (I) und (Ia),
in welchen
- R¹: für Phenyl oder Thienyl steht, wobei die oben aufgeführten Ringsysteme gegebenenfalls bis zu 2-fach, gleich oder verschieden durch Substituenten, ausgewählt aus der Gruppe Fluor, Chlor, Methyl, Trifluormethyl oder Nitro substituiert sind,
- R²: für einen Rest der Formel -OR¹² oder -NR¹³R¹⁴ steht,
worin
R¹² Wasserstoff oder (C₁-C₄)-Alkyl bedeutet,
R¹³ und R¹⁴ gleich oder verschieden sind und Wasserstoff oder Methyl bedeuten,
- R³: für Wasserstoff oder für Formyl, Cyano, Trifluormethyl oder Cyclopropyl steht,
oder für (C₁-C₃)-Alkyl steht, das gegebenenfalls durch Fluor, Chlor, Hydroxy substituiert ist, das seinerseits bis zu 3-fach durch C₁-C₃-Alkoxycarbonyl substituiert sein kann,
- R⁴: für Wasserstoff oder Methyl steht,
- D: für ein Sauerstoff- oder Schwefelatom steht,
- R⁵: für Wasserstoff, Fluor, Chlor oder für (C₁-C₃)-Alkyl steht,
und deren Salze.

Ganz besonders bevorzugt werden erfindungsgemäße Verbindungen der allgemeinen Formeln (I) und (Ia),
in welchen
- R¹: für Phenyl oder Thienyl steht, die gegebenenfalls bis zu 2-fach gleich oder verschieden durch Fluor oder Chlor substituiert sind,
- R²: für Methoxy, Ethoxy oder für n-Propoxy steht,
- R³: für Wasserstoff, Methyl oder Cyclopropyl steht,
- R⁴: für Wasserstoff steht,
- D: für ein Sauerstoff- oder Schwefelatom steht,
- R⁵: für Wasserstoff, Fluor oder Chlor steht,
und deren Salze.

Cycloalkyl mit 3 bis 6 Kohlenstoffatomen steht im Rahmen der Erfindung für Cyclopropyl, Cyclopentyl, Cyclobutyl, Cyclohexyl. Bevorzugt seien genannt: Cyclopentyl oder Cyclohexyl.

Aryl steht im allgemeinen für einen aromatischen Rest mit 6 bis 10 Kohlenstoffatomen. Bevorzugte Arylreste sind Phenyl und Naphthyl.

(C₁-C₆)-Acyl steht im Rahmen der Erfindung für einen geradkettigen oder verzweigten Acylrest mit 1 bis 6 Kohlenstoffatomen. Bevorzugt ist ein geradkettiger oder verzweigter Acylrest mit 1 bis 4 Kohlenstoffatomen. Bevorzugte Acylreste sind Acetyl und Propionyl.

(C₁-C₆)-Alkyl steht im Rahmen der Erfindung für einen geradkettigen oder verzweigten Alkylrest mit 1 bis 6 Kohlenstoffatomen. Bevorzugt ist ein geradkettiger oder verzweigter Alkylrest mit 1 bis 4 Kohlenstoffatomen.

Beispielsweise seien genannt: Methyl, Ethyl, Propyl, Isopropyl, tert.Butyl, n-Pentyl und n-Hexyl.

(C₂-C₆)-Alkenyl steht im Rahmen der Erfindung für einen geradkettigen oder verzweigten Alkenylrest mit 2 bis 6 Kohlenstoffatomen. Bevorzugt ist ein geradkettiger oder verzweigter Alkenylrest mit 3 bis 5 Kohlenstoffatomen. Beispielsweise seien genannt: Ethenyl, Propenyl, Isopropenyl, tert.Butenyl, n-Pentenyl und n-Hexenyl.

(C₁-C₆)-Alkoxy steht im Rahmen der Erfindung für einen geradkettigen oder verzweigten Alkoxyrest mit 1 bis 6 Kohlenstoffatomen. Bevorzugt ist ein geradkettiger oder verzweigter Alkoxyrest mit 1 bis 4 Kohlenstoffatomen. Beispielsweise seien genannt: Methoxy, Ethoxy, Propoxy, Isopropoxy, tert.Butoxy, n-Pentoxy und n-Hexoxy.

(C₁-C₆)-Alkoxycarbonyl steht im Rahmen der Erfindung für einen geradkettigen oder verzweigten Alkoxycarbonylrest mit 1 bis 6 Kohlenstoffatomen. Bevorzugt ist ein geradkettiger oder verzweigter Alkoxycarbonylrest mit 1 bis 4 Kohlenstoffatomen. Beispielsweise seien genannt: Methoxycarbonyl, Ethoxycarbonyl, Propoxycarbonyl, Isopropoxycarbonyl, tert.Butoxycarbonyl, n-Pentoxycarbonyl und n-Hexoxycarbonyl.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren als auch deren jeweilige Mischungen. Die Racemformen lassen sich ebenso wie die Diastereomeren in bekannter Weise in die stereoisomer einheitlichen Bestandteile trennen.

Die erfindungsgemäßen Stoffe können auch als Salze vorliegen. Im Rahmen der Erfindung sind physiologisch unbedenkliche Salze bevorzugt.

Physiologisch unbedenkliche Salze können Salze der erfindungsgemäßen Verbindungen mit anorganischen oder organischen Säuren sein. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Physiologisch unbedenkliche Salze können ebenso Metall- oder Ammoniumsalze der erfindungsgemäßen Verbindungen sein. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di-bzw. Triethylamin, Di- bzw. Triethanolamin, Dicyclohexylamin, Dimethylaminoethanol, Arginin, Lysin, Ethylendiamin oder 2-Phenylethylamin.

Die erfindungsgemäßen Verbindungen der allgemeinen Formel (I) bzw. (Ia) in welchen R⁴ für Wasserstoff steht können hergestellt werden,
indem man
[A] Aldehyde der allgemeinen Formel (II)

   R¹-CHO (II)

   in welcher
   - R¹: die oben angegebene Bedeutung hat,
   mit Amidinen oder deren Hydrochloriden der Formel (III) 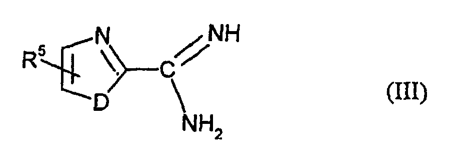 in welcher
   - R⁵ und D: die oben angegebene Bedeutung haben,
   und Verbindungen der allgemeinen Formel (IV)

   R³-CO-CH₂-CO-R² (IV)

   in welcher
   - R² und R³: die oben angegebene Bedeutung haben,
   gegebenenfalls in Gegenwart inerter organischer Lösemittel mit oder ohne Basen-bzw. Säurezusatz umsetzt,
   oder
[B] Verbindungen der allgemeinen Formel (V) 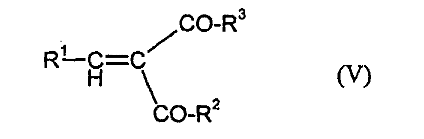 in welcher
   - R¹, R² und R³: die oben angegebene Bedeutung haben,
   mit Amidinen der allgemeinen Formel (III) 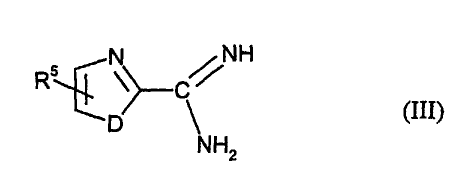 in welcher
   - R⁵ und D: die oben angegebene Bedeutung haben,
   gegebenenfalls in Gegenwart inerter organischer Lösemittel bei Temperaturen zwischen 20°C und 150°C mit oder ohne Basen- oder Säurezusatz umsetzt,
   oder
[C] Aldehyde der allgemeinen Formel (II)

   R¹-CHO (II)
in welcher
- R¹: die oben angegebene Bedeutung hat,
mit Verbindungen der allgemeinen Formel (VI) 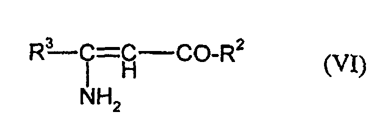 in welcher
- R² und R³: die oben angegebene Bedeutung haben,
und Amidinen der allgemeinen Formel (III) wie oben-beschrieben umsetzt.

Die erfindungsgemäßen Verfahren können durch folgende Formelschemata beispielhaft erläutert werden:

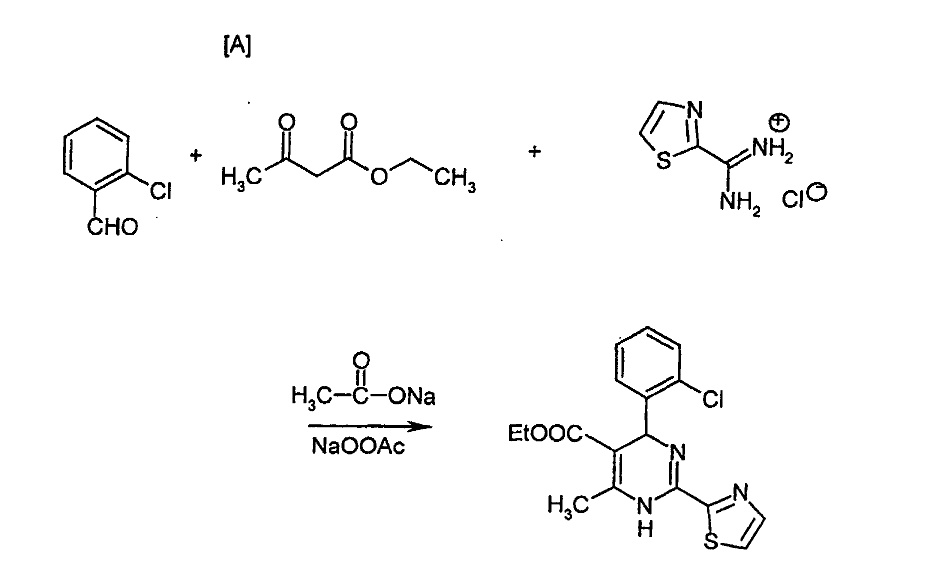

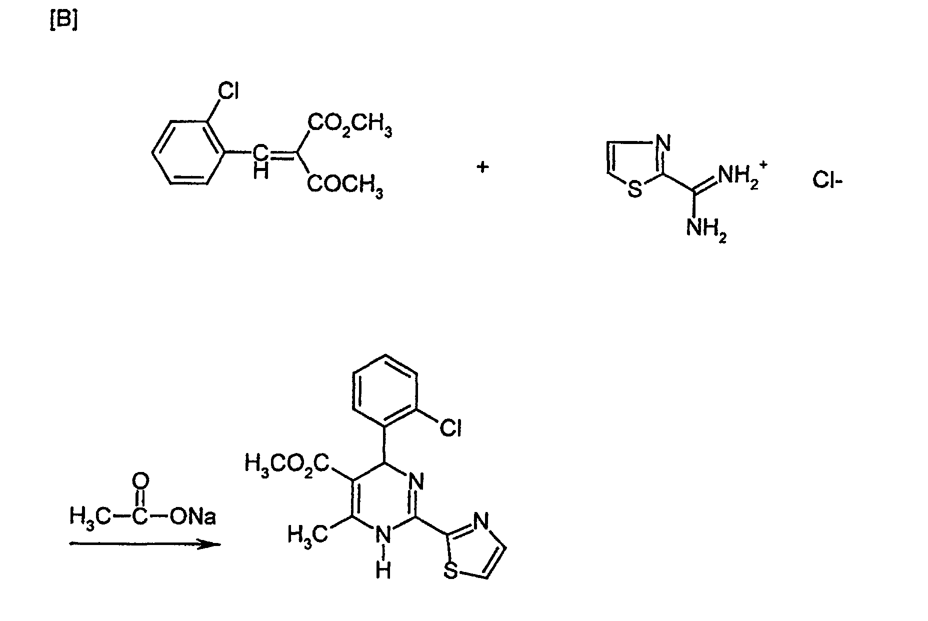

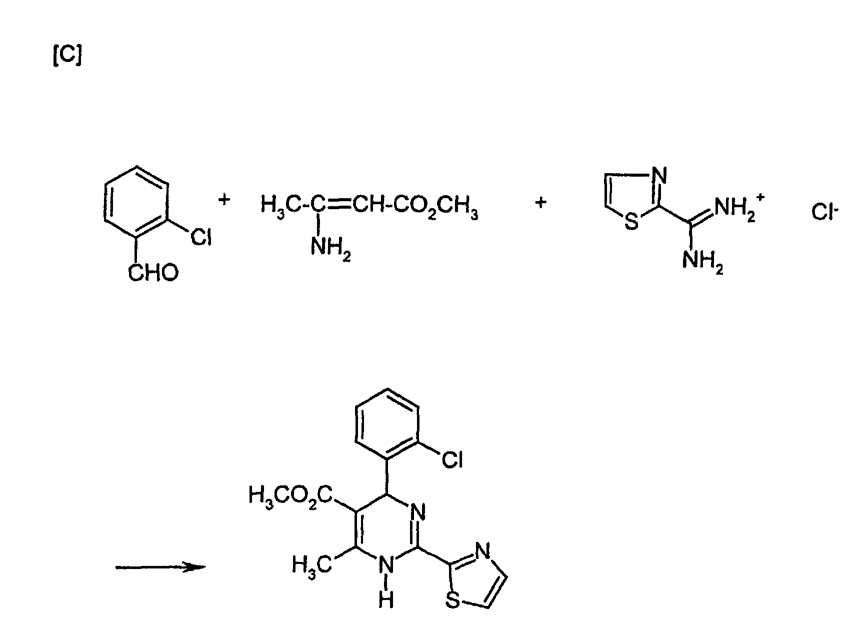

Für alle Verfahrensvarianten A, B und C kommen als Lösemittel alle inerten organischen Lösemittel in Frage. Hierzu gehören vorzugsweise Alkohole wie Ethanol, Methanol, Isopropanol, Ether wie Dioxan, Diethylether, Tetrahydrofuran, Glykolmonomethylether, Glykoldimethylether oder Eisessig, Dimethylformamid, Dimethylsulfoxid, Acetonitril, Pyridin und Hexamethylphosphorsäuretriamid.

Die Reaktionstemperaturen können in einem größeren Bereich variiert werden. Im allgemeinen arbeitet man zwischen 20 und 150°C, vorzugsweise jedoch bei der Siedetemperatur des jeweiligen Lösemittels.

Die Umsetzung kann bei Normaldruck, aber auch bei erhöhtem Druck durchgeführt werden. Im allgemeinen arbeitet man unter Normaldruck.

Die Umsetzung kann mit oder ohne Basen- bzw. Säurezusatz durchgeführt werden, es hat sich jedoch gezeigt, daß eine Umsetzung im Sinne der Erfindung vorzugsweise in Gegenwart von schwächeren Säuren wie z.B. Essigsäure oder Ameisensäure stattfindet.

Die als Ausgangsstoffe verwendeten Aldehyde der allgemeinen Formel (II) sind bekannt oder können nach literaturbekannten Methoden darstellt werden [vgl. T.D. Harris und G.P. Roth, J. Org. Chem. 44, 146 (1979), Deutsche Offenlegungsschrift 2 165 260, Juli 1972, Deutsche Offenlegungsschrift 2 401 665, Juli 1974, Mijano et al., Chem. Abstr. 59, (1963), 13 929 c, E. Adler und H.-D. Becker, Chem. Scand. 15, 849 (1961), E.P. Papadopoulos, M. Mardin und Ch. Issidoridis, J. Org. Chem. Soc. 78, 2543 (1956)].

Die als Ausgangsstoffe verwendeten Amidine der allgemeinen Formel (III) sind teilweise bekannt oder können nach literaturbekannten Methoden hergestellt werden [vgl. Houben-Weyl, Methoden der organischen Chemie, Vol. 11/2, Seite 38 ff (1958); R.L. Shoiner und F.W. Neumann, Chem. Review 35, 351 (1944)].

Die als Ausgangsstoffe verwendeten β-Ketocarbonsäureester der allgemeinen Formel (IV) sind bekannt oder können nach literaturbekannten Methoden dargestellt werden [z.B. D. Borrmann, "Umsetzung von Diketen mit Alkoholen, Phenolen und Mercaptanen", in Houben-Weyl, Methoden der organischen Chemie, Vol. VII/4, 230 ff (1968); Y. Oikawa, K. Sugano und O. Yonemitsu, J. Org. Chem. 43, 2087 (1978)].

Die als Ausgangsstoffe verwendeten Yliden-β-ketoester der Formel (V) können nach literaturbekannten Methoden dargestellt werden [vgl. G. Jones, "The Knoevenagel Condensation", in Organic Reactions, Vol. XV, 204 ff. (1967)].

Die als Ausgangsstoffe verwendeten Enaminocarbonsäureester der (VI) sind bekannt oder können nach literaturbekannten Methoden dargestellt werden [vgl. A.C. Cope, J. Am. Chem. Soc. 67, 1017 (1945)].

Die antivirale Wirkung der erfindungsgemäßen Verbindungen wurden in Anlehnung an die von Sells et al. (M.A. Sells, M.-L. Chen, and G. Acs (1987) Proc. Natl. Acad. Sci. 84, 1005 - 1009) und Korba et al. (B.E. Korba and J.L. Gerin (1992) Antiviral Research 19, 55-70) beschriebenen Methoden durchgeführt.

Die antiviralen Tests wurden in 96-well-Mikrotiterplatten durchgeführt. Die erste vertikale Reihe der Platte erhielt nur Wachstumsmedium und HepG2.2.15-Zellen. Sie diente als Viruskontrolle.

Stammlösungen der Testverbindungen (50 mM) wurden zunächst in DMSO gelöst. Weitere Verdünnungen wurden in Wachstumsmedien der Hepatitis B-Virus produzierenden HepG2.2.15-Zellen hergestellt. Die erfindungsgemäßen Verbindungen wurden in der Regel in einer Testkonzentration von 100 µM (1. Testkonzentration) jeweils in die zweite vertikale Testreihe der Mikrotiterplatte pipettiert und anschließend in 2-er Schritten 2¹⁰ fach in Wachstumsmedium plus 2% foetales Kälberserum, verdünnt (Volumen 25 µl).
Jeder Napf der Mirotiterplatte erhielt dann 225 µl einer HepG2.2.15 Zellsuspension (5 x 10⁴ Zellen/ml) in Wachstumsmedium plus 2% fötales Kälberserum.
Der Testansatz wurde 4 Tage, 37° Celsius, 5 % CO₂, inkubiert.
Anschließend wurde der'Überstand abgesaugt und verworfen, und die Näpfe erhielten 225 µl frisch zubereitetes Wachtumsmedium. Die erfindungsgemäßen Verbindungen wurden jeweils erneut als 10-fach konzentrierte Lösung in einem Volumen von 25 µl zugefügt. Die Ansätze wurden weitere 4 Tage inkubiert.
Vor der Ernte der Überstände zur Bestimmung des antiviralen Effektes wurden die HepG2.2.15-Zellen lichtmikroskopisch oder mittels biochemischer Nachweisverfahren (z.B. Alamar Blue Färbung oder Trypanblau-Färbung) auf zytotoxische Veränderungen untersucht.
Anschließend wurden die Überstände geerntet und mittels Vakuum auf mit Nylonmembran bespannten 96-Napf Dot Blot Kammern (entsprechend den Angaben der Hersteller) gesogen.

### Zytotoxizitätsbestimmung

Substanzinduzierte zytotoxische oder zytostatische Veränderungen der HepG2.2.15-Zellen wurden z.B. lichtmikroskopisch als Änderungen der Zellmorphologie ermittelt. Derartige Substanz-induzierte Veränderungen der HepG2.2.15-Zellen im Vergleich zu unbehandelten Zellen wurden z.B. als Zellyse, Vakuolisierung oder veränderter Zellmorphologie sichtbar. 50 % Zytotoxizität (Tox.-50) bedeuten, daß 50 % der Zellen eine der entsprechenden Zellkontrolle vergleichbare Morphologie aufweisen.

Die Verträglichkeit einiger der erfindungsgemäßen Verbindungen wurde zusätzlich auf anderen Wirtszellen wie z.B. HeLa-Zellen, primäre periphere Blutzellen des Menschen oder transformierte Zellinien wie H-9 Zellen, ausgetestet.
Es konnten keine zytotoxischen Veränderungen bei Konzentrationen der erfindungsgemäßen Verbindungen von >10µM festgestellt werden.

### Bestimmung der antiviralen Wirkung

In Kürze: Nach Transfer der Überstände auf die Nylon Membran der Blot Apparatur (s.o.) wurden die Überstände der HepG2.2.15-Zellen denaturiert (1.5 M NaCl/ 0.5 N NaOH), neutralisiert (3 M NaCl/ 0.5 M Tris HCl, pH 7.5) und gewaschen (2 x SSC). Anschließend wurde die DNA durch Inkubation der Filter bei 120 °C, 2 - 4 Stunden, an die Membran gebacken.

### Hybridisierung der DNA

Der Nachweis der viralen DNA von den behandelten HepG2.2.15-Zellen auf den Nylonfiltern wurde in der Regel mit nichtradioaktiven, Digoxigenin-markierten Hepatitis B spezifischen DNA-Sonden durchgeführt, die jeweils nach Angabe des Herstellers mit Digoxigenin markiert, gereinigt und zur Hybidisierung eingesetzt wurden.

Kurz: die Prähybidisierung und Hybidisierung erfolgte in 5 x SSC, 1 x Blockierungsreagenz, 0.1% N-Lauroylsarcosin, 0.02 % SDS und 100 µg Sperma DNA des Herings. Die Prähybridisierung erfolgte 30 Minuten bei 60 °C, die spezifische Hybridisierung mit 20 - 40 ng/ml der digoxigenierten, denaturierten HBV-spezifischen DNA (14 Stunden, 60 °C). Anschließend wurden die Filter gewaschen.

### Nachweis der HBV DNA durch Digoxigenin Antikörper

Der immunologische Nachweis der Digoxigenin markierten DNA erfolgte nach Angaben des Herstellers.
Kurz: die Filter wurden gewaschen und in einem Blockierungsreagenz (nach Angabe des Herstellers) prähybridisiert. Anschließend wurde mit einem Anti-DIG-Antikörper, der mit alkalischer Phosphatase gekoppelt war, 30 Minuten hybridisiert. Nach einem Waschschritt wurde das Substrat der alkalischen Phosphatase, CSPD, zugefügt, 5 Minuten mit den Filtern inkubiert, anschließend in Plastikfolie eingepackt und weitere 15 Minuten bei 37 °C inkubiert. Die Chemilumineszenz der Hepatitis B spezifischen DNA Signale wurde über eine Exposition der Filter auf einem Röntgenfilm sichtbar gemacht (Inkubation je nach Signalstärke: 10 Minuten bis 2 Stunden). Die halbmaximale Hemmkonzentration (IC-50, inhibitorische Konzentration 50 %) wurde als die Konzentration bestimmt, bei der gegenüber einer unbehandelten Probe die Hepatitis B spezifische Bande durch die erfindungsgemäße Verbindung um 50 % reduziert wurde.

Die Behandlung der Hepatits B Virus-produzierenden HepG2.2.15 Zellen mit den erfindungsgemäßen Verbindungen führte überraschenderweise zu einer Reduktion viraler DNA im Zellkulturüberstand, die von den Zellen in Form von Virionen in den Zellkulturüberstand ausgeschleust wird.

Die erfindungsgemäßen Verbindungen zeigen ein neue, nicht vorhersehbare und wertvolle Wirkung gegen Viren. Sie sind überraschenderweise antiviral gegen Hepatitis B (HBV) wirksam und sind somit zur Behandlung von virusinduzierten Erkrankungen, insbesondere von akut und chronisch persistenten Virusinfektionen des HBV geeignet. Ein chronische Viruserkrankung, hervorgerufen durch das HBV, kann zu unterschiedlich schweren Krankheitsbildern führen; bekanntermaßen führt die chronische Hepatitis B Virusinfektion in vielen Fällen zur Leberzirrhose und/oder zum hepatozellulären Karzinom.

Als Indikationsgebiete für die erfindungsgemäß verwertbaren Verbindungen können beispielsweise genannt werden:

Die Behandlung von akuten und chronischen Virusinfektionen, die zu einer infektiösen Hepatitis führen können, beispielsweise die Infektionen mit Hepatitis B Viren. Besonders bevorzugt ist die Behandlung von chronischen Hepatitis-B Infektionen und die Behandlung von akuter Hepatitis-B Virusinfektion.

Zur vorliegenden Erfindung gehören pharmazeutische Zubereitungen, die neben nichttoxischen, inerten pharmazeutisch geeigneten Trägerstoffen eine oder mehrere Verbindungen der Formeln (I) und (Ia) enthalten oder die aus einem oder mehreren Wirkstoffen der Formel (I) bestehen, sowie Verfahren zur Herstellung dieser Zubereitungen.

Die Wirkstoffe der Formeln (I) und (Ia) sollen in den oben aufgeführten pharmazeutischen Zubereitungen in einer Konzentration von etwa 0,1 bis 99,5 Gew.-%, vorzugsweise von etwa 0,5 bis 95 Gew.-% der Gesamtmischung, vorhanden sein.

Die oben aufgeführten pharmazeutischen Zubereitungen können außer den Verbindungen der Formeln (I) und (Ia) auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 1 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 1 bis etwa 80, insbesondere 1 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels, sowie dem Zeitraum bzw. Intervall, innerhalb welchem die Verabreichung erfolgt.

### Ausgangsverbindungen

### Beispiel I

2-Amidino-thiazol-Hydrochlorid

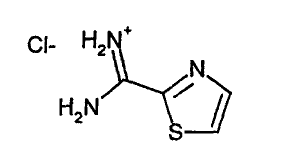

4,5 g Natrium (0,2 mol) werden portionsweise zu 220 ml MeOH gegeben. Nach Bildung des Methanolats werden dazu 22,9 g (0,21 mol) 2-Cyano-thiazol (J. Chem. Soc. C, 1967, S. 517) gegeben und zwei Tage bei RT gerührt. Es wird von anorganischen Salzen abgesaugt, eingeengt, mit wenig Aceton kristallisiert und das Produkt abgesaugt. Man erhält 17,4 g (51 %) 2-Amidino-thiazol-hydrochlorid.

Fp: 188° C, Zers.

R_{f}: 0,26 (EE/MeOH/H₂O/konz. NH₃= 6 : 3 : 0,5 : 0,05)

### Herstellungsbeispiele

### Beispiel 1

4-(2-Chlorphenyl)-6-methyl-2-(thiazolyl-2)-1,4-dihydropyrimidin-5-carbonsäure methylester

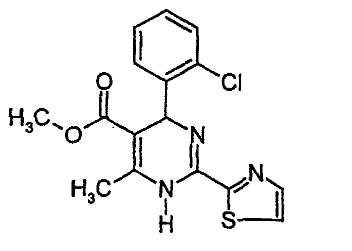

320 mg (2 mmol) 2-Amidino-thiazol-hydrochlorid, 470 mg (2 mmol) 2-Chlorbenzyliden-acetessigäure-methylester und 200 mg (2,4 mmol) Natriumacetat werden in 10 ml Ethanol 16 h am Rückfluß gekocht. Es wird eingeengt, mit verd. NaOH alkalisch gestellt und mit EE ausgeschüttelt. Nach Reinigung über Kieselgel (Cyclohexan: EE = 7:3) erhält man 350 mg (32 %) Substanz.
R_{f} = 0,23 (Cyclohexan/EE = 7:3)

### Beispiel 2

4-(4-Chlorphenyl)-6-methyl-2-(thiazolyl-2)-1,4-dihydropyrimidin-5-carbonsäure methylester

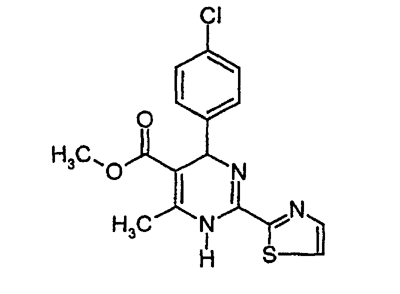

270 mg (1,7 mmol) 2-Amidino-thiazol, 200 mg (1,7 mmol) Acetessigsäuremethylester, 230 mg (1,7 mmol) 4-Chlorbenzaldehyd und 165 mg (2 mmol) Natriumacetat werden in 11 ml Ethanol 16 h am Rückfluß gekocht. Aufarbeitung wie unter Beispiel 1 ergibt 350 mg (31 %) amorphe Substanz.
R_{f}= 0,20 (Cyclohexan/EE = 7:3)

Analog der Vorschrift des Beispiels 2 werden die in Tabelle 1 aufgeführten Verbindungen hergestellt:

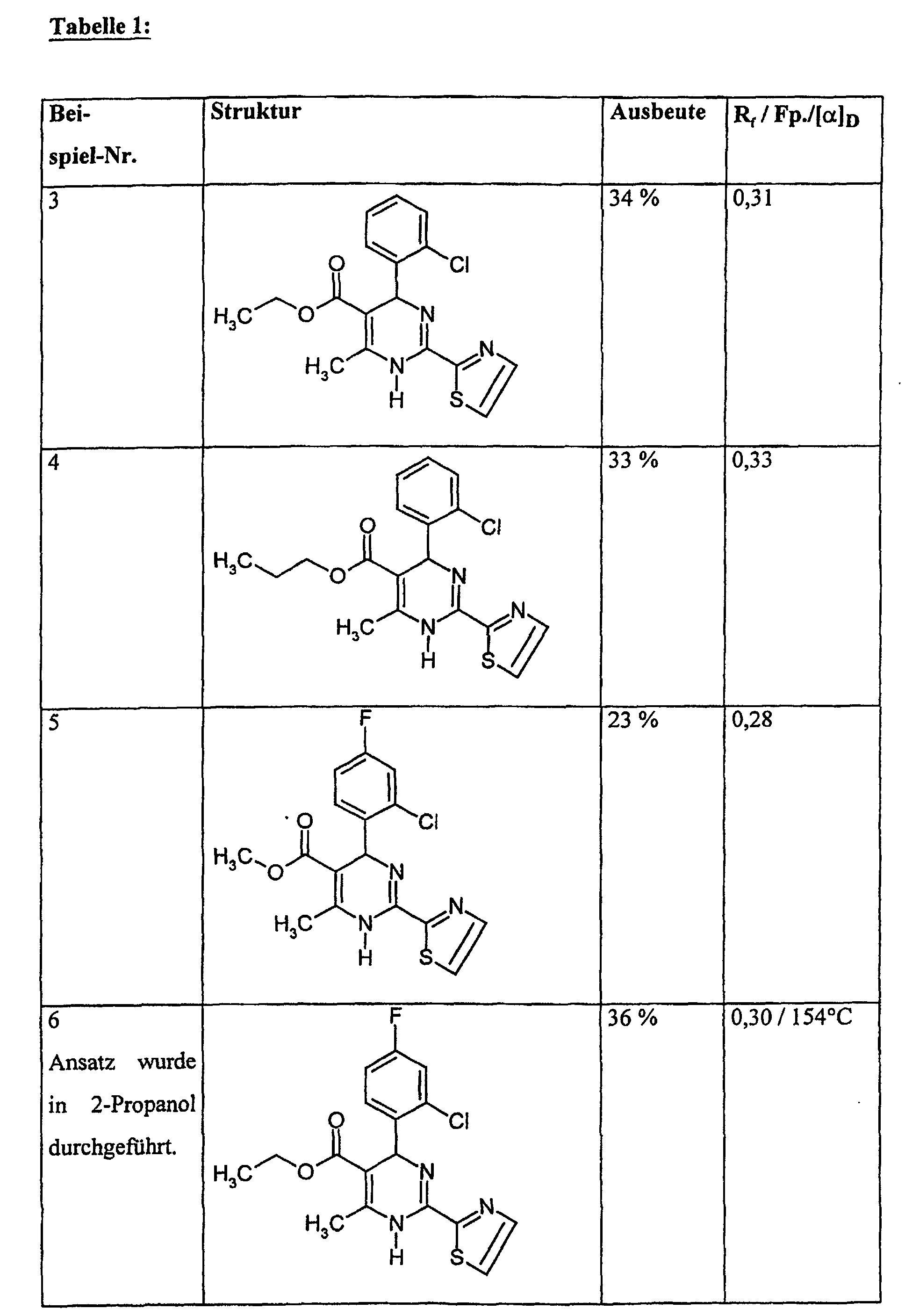

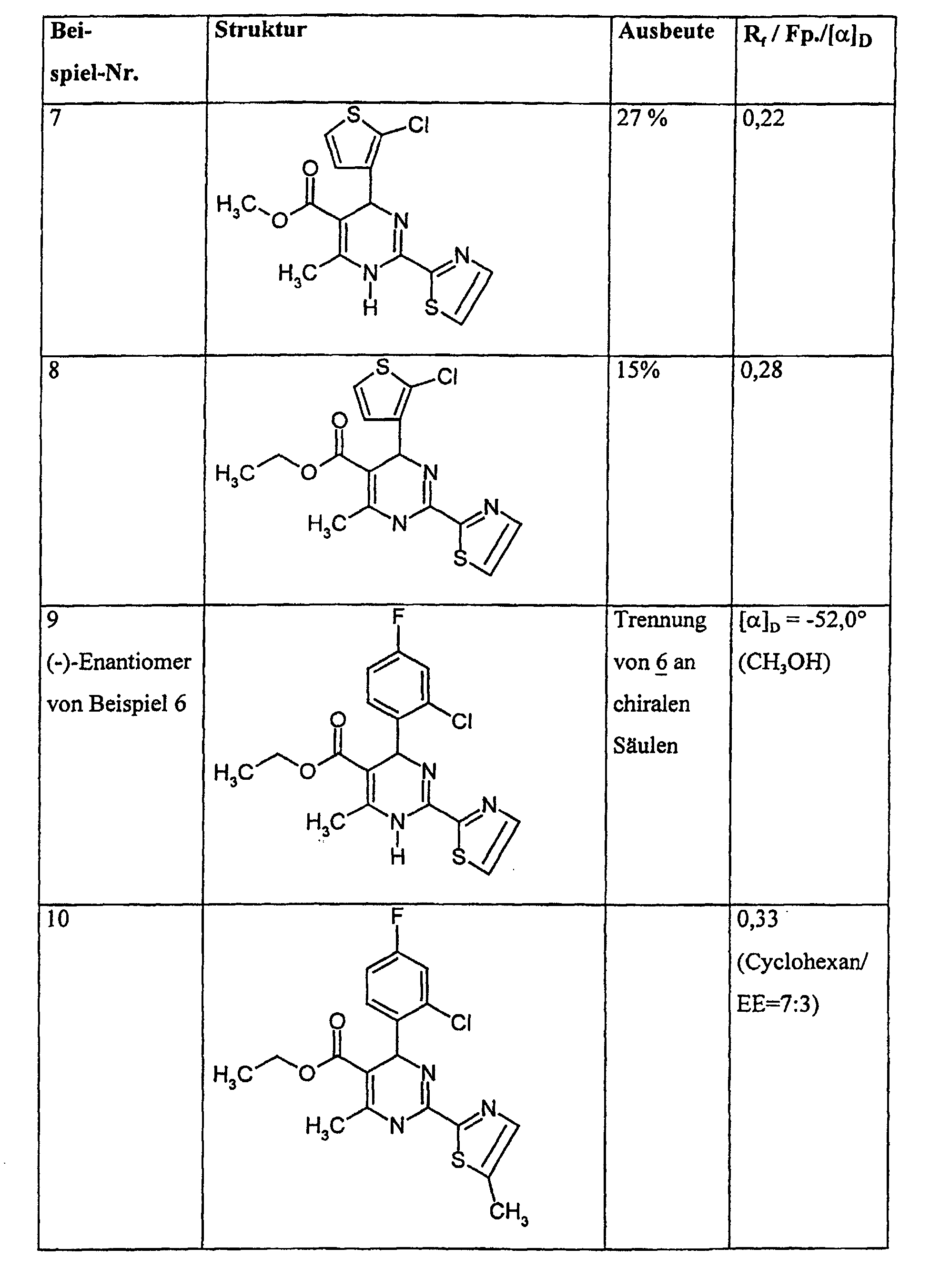

### Wanderungsgeschwindigkeiten

- R_{f}=: die Entfernung einer Substanz vom Ausgangspunkt dividiert durch die Entfernung der Lösungsmittelfront ebenfalls vom Ausgangspunkt
- Fp. =: Schmelzpunkt
- [α]_{D}0 =: Drehwert
- EE=: Essigsäureethylester

## Patentansprüche

1. Dihydropyrimidine der allgemeinen Formel (I) 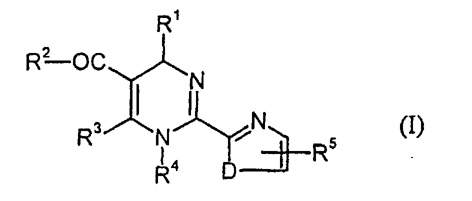 bzw. deren isomeren Form (Ia) 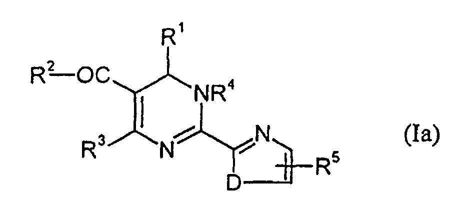 in welchen
R¹ für Phenyl, Furyl, Thienyl, Pyridyl, Cycloalkyl mit 3 bis 6 Kohlenstoffatomen oder für einen Rest der Formel 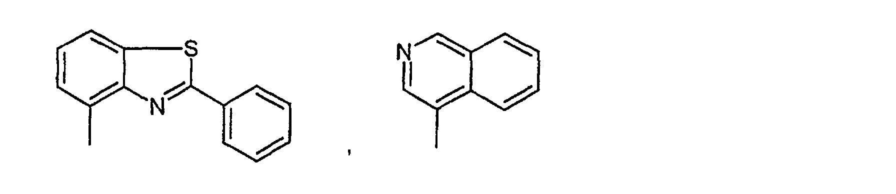 oder 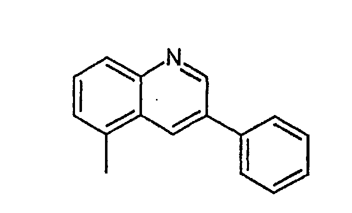 steht,
wobei die oben aufgeführten Ringsysteme gegebenenfalls einfach oder mehrfach, gleich oder verschieden durch Substituenten, ausgewählt aus der Gruppe Halogen, Trifluormethyl, Nitro, Cyano, Trifluormethoxy, Carboxyl, Hydroxyl, (C₁-C₆)-Alkoxy, (C₁-C₆)-Alkoxycarbonyl und (C₁-C₆)-Alkyl substituiert sind, das seinerseits durch Aryl mit 6 bis 10 Kohlenstoffatomen oder Halogen substituiert sein kann,
und/oder die aufgeführten Ringsysteme gegebenenfalls durch Gruppen der Formel -S-R⁶, NR⁷R⁸, CO-NR⁹R¹⁰, SO₂-CF₃ und -A-CH₂-R¹¹ substituiert sind,
worin
R⁶ Phenyl bedeutet, das gegebenenfalls durch Halogen substituiert ist,
R⁷, R⁸, R⁹ und R¹⁰ gleich oder verschieden sind und Wasserstoff, Phenyl, Hydroxy-substituiertes Phenyl, Hydroxy, (C₁-C₆)-Acyl oder (C₁-C₆)-Alkyl bedeuten, das seinerseits durch Hydroxy, (C₁-C₆)-Alkoxycarbonyl, Phenyl oder Hydroxy substituiertes Phenyl substituiert sein kann,
A einen Rest O, S, SO oder SO₂ bedeutet,
R¹¹ Phenyl bedeutet, das gegebenenfalls ein- bis mehrfach, gleich oder verschieden durch Substituenten, ausgewählt aus der Gruppe Halogen, Nitro, Trifluormethyl, (C₁-C₆)-Alkyl und (C₁-C₆)-Alkoxy substituiert ist,
R² für einen Rest der Formel -OR¹² oder -NR¹³R¹⁴ steht,
worin
R¹² Wasserstoff, (C₁-C₆)-Alkoxycarbonyl oder einen geradkettigen, verzweigten oder cyclischen, gesättigten oder ungesättigten (C₁-C₈)- Kohlenwasserstoffrest bedeutet, der gegebenenfalls eine oder zwei gleiche oder verschiedene Heterokettenglieder aus der Gruppe O, CO, NH, -N-(C₁-C₄)-Alkyl,-N-((C₁-C₄)-Alkyl)₂, S oder SO₂ enthält und der gegebenenfalls substituiert ist durch Halogen, Nitro, Cyano, Hydroxy, Aryl mit 6 bis 10 Kohlenstoffatomen oder Aralkyl mit 6 bis 10 Kohlenstoffatomen, Heteroaryl oder einer Gruppe der Formel -NR¹⁵R¹⁶,
worin
R¹⁵ und R¹⁶ gleich oder verschieden sind und Wasserstoff, Benzyl oder (C₁-C₆)-Alkyl bedeuten,
R¹³ und R¹⁴ gleich oder verschieden sind und Wasserstoff, (C₁-C₆)-Alkyl oder Cycloalkyl mit 3 bis 6 Kohlenstoffatomen bedeuten,
R³ für Wasserstoff,Amino oder
für einen Rest der Formel 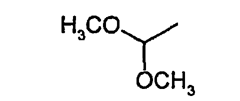 steht, oder
für Formyl, Cyano oder Trifluormethyl steht, oder
für einen geradkettigen, verzweigten oder cyclischen, gesättigten oder ungesättigten Kohlenwasserstoffrest mit bis zu 8 Kohlenstoffatomen steht, der gegebenenfalls ein- oder mehrfach, gleich oder verschieden durch Aryloxy mit 6 bis 10 Kohlenstoffatomen, Azido, Cyano, Hydroxy, Carboxyl, (C₁-C₆)-Alkoxycarbonyl, einen 5- bis 7-gliedrigen heterocyclischen Ring, (C₁-C₆)-Alkylthio, (C₁-C₆)-Alkoxy substituiert ist, das seinerseits durch Azido oder Amino substituiert sein kann,
und/oder durch Triazolyl substituiert ist, das seinerseits bis zu 3-fach durch (C₁-C₆)-Alkoxycarbonyl substituiert sein kann,
und/oder durch Gruppen der Formeln -OSO₂-CH₃ oder (CO)ₐ-NR¹⁷R¹⁸ substiutiert sein kann,
worin
a eine Zahl 0 oder 1 bedeutet,
R¹⁷ und R¹⁸ gleich oder verschieden sind und Wasserstoff oder Aryl, Aralkyl mit 6 bis 10 Kohlenstoffatomen bedeuten,
oder (C₁-C₆)-Alkyl bedeuten, das gegebenenfalls durch (C₁-C₆)-Alkoxycarbonyl, Hydroxyl, Phenyl oder Benzyl substituiert ist, wobei Phenyl oder Benzyl gegebenenfalls ein- oder mehrfach, gleich oder verschieden durch Hydroxy, Carboxyl, (C₁-C₆)-Alkyl oder (C₁-C₆)-Alkoxy substituiert sind,
oder (C₁-C₆)-Alkyl gegebenenfalls durch Gruppen der Formeln NH-CO-CH₃ oder NH-CO-CF₃ substituiert ist,
R⁴ für Wasserstoff, (C₁-C₄)-Alkyl, Acetyl oder Benzoyl steht,
D für ein Sauerstoff- oder Schwefelatom steht,
R⁵ für Wasserstoff, Halogen oder für geradkettiges oder verzweigtes Alkyl mit bis zu 6 Kohlenstoffatomen steht,
und deren Salze.

2. Dihydropyrimidine gemäß Anspruch 1,
in welchen
R¹ für Phenyl, Furyl, Thienyl, Pyridyl, Cyclopentyl oder Cyclohexyl steht, wobei die oben aufgeführten Ringsysteme gegebenenfalls ein-oder 2-fach gleich oder verschieden durch Substituenten, ausgewählt aus der Gruppe Halogen, Trifluormethyl, Nitro, SO₂-CF₃, Methyl, Cyano, Trifluormethoxy, Carboxyl, Methoxycarbonyl oder Resten der Formel -CO-NH-CH₂-C(CH₃)₃, -CO-NH(CH₂)₂OH, -CO-NH-CH₂-C₆H₅, -CO-NH-C₆H₅, -CO-NH-(pOH)-C₆H₄, -O-CH₂-C₆H₅ oder -SpCl-C₆H₄ substituiert sind,
R² für einen Rest der Formel -OR¹² oder -NR¹³R¹⁴ steht,
worin
R¹² Wasserstoff, (C₁-C₄)-Alkenyl oder (C₁-C₄)-Alkyl bedeutet, das gegebenenfalls durch Pyridyl, Cyano, Phenoxy, Benzyl oder durch einen Rest der Formel -NR¹⁵R¹⁶ substituiert ist,
worin
R¹⁵ und R¹⁶ gleich oder verschieden sind und Wasserstoff, Methyl oder Ethyl bedeuten,
R¹³ und R¹⁴ gleich oder verschieden sind und Wasserstoff, Methyl, Ethyl oder Cyclopropyl bedeuten,
R³ für Wasserstoff oder einen Rest der Formel 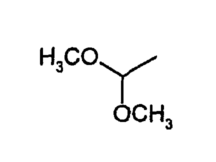 steht,
oder
für Formyl, Cyano, Trifluormethyl oder Cyclopropyl steht, oder
für (C₁-C₄)-Alkyl steht, das gegebenenfalls durch Reste der Formeln -SO₂CH₃, -NH-CO-CH₃, -NH-CO-CF₃, Fluor, Chlor, (C₁-C₃)-Alkoxycarbonyl oder Hydroxy, substituiert ist,
und/oder Alkyl gegebenenfalls durch Gruppen der Formeln -OSO₂-CH₃ oder (CO)ₐ-NR¹⁷R¹⁸ substituiert ist,
worin
a eine Zahl 0 oder 1 bedeutet,
R¹⁷ und R¹⁸ gleich oder verschieden sind und Wasserstoff, Phenyl oder Benzyl bedeuten, oder
C₁-C₄-Alkyl bedeuten, das gegebenenfalls durch (C₁-C₄)-Alkoxycarbonyl, Hydroxyl, Phenyl oder Benzyl substituiert ist, wobei Phenyl oder Benzyl gegebenenfalls ein- oder mehrfach gleich oder verschieden durch Hydroxy, Carboxy, (C₁-C₄)-Alkyl oder (C₁-C₄)-Alkoxy substituiert sind,
und/oder (C₁-C₄)-Alkyl gegebenenfalls durch Reste der Formeln -NH-CO-CH₃ oder -NH-CO-CF₃ substituiert ist,
oder
R⁴ für Wasserstoff, Methyl, Ethyl oder Acetyl steht,
D für ein Sauerstoff- oder Schwefelatom steht,
R⁵ für Wasserstoff, Halogen oder für C₁-C₄)-Alkyl steht,
und deren Salze.

3. Dihydropyrimidine gemäß Anspruch 1 oder 2,
in welchen
R¹ für Phenyl oder Thienyl steht, wobei die oben aufgeführten Ringsysteme gegebenenfalls bis zu 2-fach, gleich oder verschieden durch Substituenten, ausgewählt aus der Gruppe Fluor, Chlor, Methyl, Trifluormethyl oder Nitro substituiert sind,
R² für einen Rest der Formel -OR¹² oder -NR¹³R¹⁴ steht,
worin
R¹² Wasserstoff oder (C₁-C₄)-Alkyl bedeutet,
R¹³ und R¹⁴ gleich oder verschieden sind und Wasserstoff oder Methyl bedeuten,
R³ für Wasserstoff oder für Formyl, Cyano, Trifluormethyl oder Cyclopropyl steht,
oder für (C₁-C₃)-Alkyl steht, das gegebenenfalls durch Fluor, Chlor, Hydroxy substituiert ist, das seinerseits bis zu 3-fach durch C₁-C₃-Alkoxycarbonyl substituiert sein kann,
R⁴ für Wasserstoff oder Methyl steht,
D für ein Sauerstoff- oder Schwefelatom steht,
R⁵ für Wasserstoff, Fluor, Chlor oder für (C₁-C₃)-Alkyl steht,
und deren Salze.

4. Dihydropyrimidine gemäß einem der Ansprüche 1 bis 3,
in welchen
R¹ für Phenyl oder Thienyl steht, die gegebenenfalls bis zu 2-fach gleich oder verschieden durch Fluor oder Chlor substituiert sind,
R² für Methoxy, Ethoxy oder für n-Propoxy steht,
R³ für Wasserstoff, Methyl oder Cyclopropyl steht,
R⁴ für Wasserstoff steht,
D für ein Sauerstoff- oder Schwefelatom steht,
R⁵ für Wasserstoff, Fluor oder Chlor steht,
und deren Salze.

5. Verfahren zur Herstellung von Dihydropyrimidine der allgemeinen Formel (I) oder (Ia) gemäß einem der Ansprüche 1 bis 4 in welchen R⁴ für Wasserstoff steht, **dadurch gekennzeichnet, daß**
[A] Aldehyde der allgemeinen Formel (II)
R¹-CHO (II)
in welcher
R¹ die oben angegebene Bedeutung hat,
mit Amidinen oder deren Hydrochloriden der Formel (III) 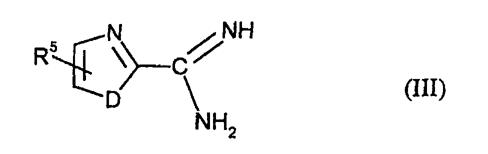 in welcher
R⁵ und D die oben angegebene Bedeutung haben,
und Verbindungen der allgemeinen Formel (IV)
R³-CO-CH₂-CO-R² (IV)
in welcher
R² und R³ die oben angegebene Bedeutung haben,
oder
[B] Verbindungen der allgemeinen Formel (V) 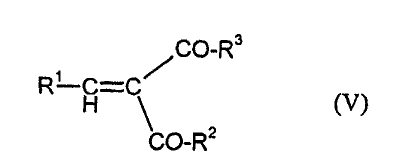 in welcher
R¹, R² und R³ die oben angegebene Bedeutung haben,
mit Amidinen der allgemeinen Formel (III) 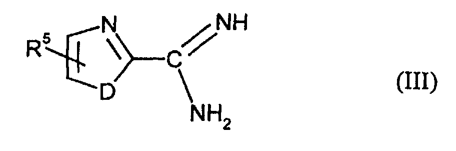 in welcher
R⁵ und D die oben angegebene Bedeutung haben,
oder
[C] Aldehyde der allgemeinen Formel (II)
R¹-CHO (II)
in welcher
R¹ die oben angegebene Bedeutung hat,
mit Verbindungen der allgemeinen Formel (VI) 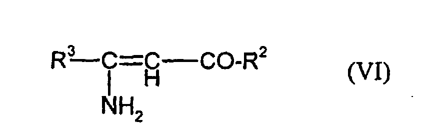 in welcher
R² und R³ die oben angegebene Bedeutung haben,
und Amidinen der allgemeinen Formel (III) umsetzt.

6. Arzneimittel, enthaltend mindestens eine Verbindung der allgemeinen Formel (I) oder (Ia) gemäß einem der Ansprüche 1 bis 4 und gegebenenfalls enthaltend weitere pharmazeutische Wirkstoffe.

7. Verfahren zur Herstellung von Arzneimittel, **dadurch gekennzeichnet, daß** mindestens eine Verbindung der allgemeinen Formel (I) oder (Ia) gemäß einem der Ansprüche 1 bis 4, gegebenenfalls unter Verwendung von üblichen Hilfs- und Trägerstoffen in eine geeignete Applikationsform überführt wird.

8. Verwendung von Verbindungen der allgemeinen Formel (I) oder (Ia) gemäß einem der Ansprüche 1 bis 4 zur Herstellung eines Arzneimittels.

9. Verwendung gemäß Anspruch 8 zur Herstellung eines Arzneimittels zur Behandlung von aktuten oder chronischen Viruserkrankungen.

10. Verwendung gemäß Anspruch 8 oder 9 zur Herstellung eines Arzneimittels zur Behandlung von akuten oder chronischen Hepatitis-B-Infektionen.

## Claims

1. Dihydroxypyrimidines of the general formula (I) 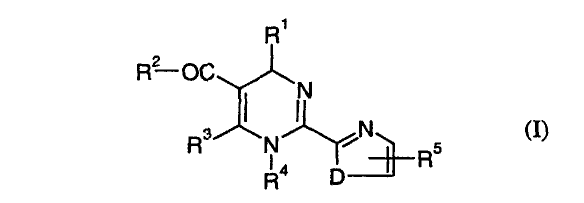 or their isomeric form (Ia) 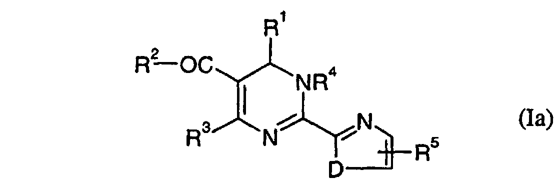 in which
R¹ represents phenyl, furyl, thienyl, pyridyl, cycloalkyl having 3 to 6 carbon atoms or a radical of the formula 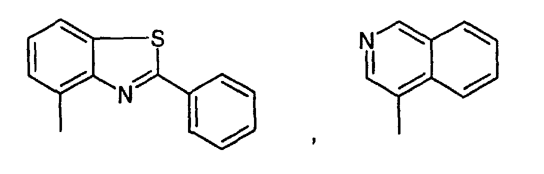 or 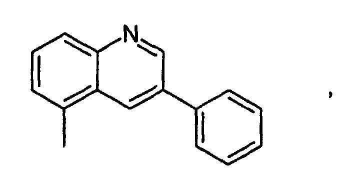 where the abovementioned ring systems are optionally monosubstituted or polysubstituted, in an identical or different manner, by substituents selected from the group consisting of halogen, trifluoromethyl, nitro, cyano, trifluoromethoxy, carboxyl, hydroxyl, (C₁-C₆)-alkoxy, (C₁-C₆)-alkoxycarbonyl and (C₁-C₆)-alkyl, which for its part can be substituted by aryl having 6 to 10 carbon atoms or halogen,
and/or the ring systems mentioned are optionally substituted by groups of the formula -S-R⁶, NR⁷R⁸, CO-NR⁹R¹⁰, SO₂-CF₃ and -A-CH₂-R¹¹,
in which
R⁶ denotes phenyl which is optionally substituted by halogen,
R⁷, R⁸, R⁹ and R¹⁰ are identical or different and denote hydrogen, phenyl, hydroxy-substituted phenyl, hydroxyl, (C₁-C₆)-acyl or (C₁-C₆)-alkyl, which for its part can be substituted by hydroxyl, (C₁-C₆)-alkoxycarbonyl, phenyl or hydroxy-substituted phenyl,
A denotes a radical O, S, SO or SO₂,
R¹¹ denotes phenyl which is optionally mono- to polysubstituted, in an identical or different manner, by substituents selected from the group consisting of halogen, nitro, trifluoromethyl, (C₁-C₆)-alkyl and (C₁-C₆)-alkoxy,
R² represents a radical of the formula -OR¹² or -NR¹³R¹⁴,
in which
R¹² denotes hydrogen, (C₁-C₆)-alkoxycarbonyl or a straight-chain, branched or cyclic, saturated or unsaturated (C₁-C₈)-hydrocarbon radical which optionally contains one or two identical or different hetero chain members from the group consisting of O, CO, NH, -N-(C₁-C₄)-alkyl, -N-((C₁-C₄)-alkyl)₂, S and SO₂ and which is optionally substituted by halogen, nitro, cyano, hydroxyl, aryl having 6 to 10 carbon atoms or aralkyl having 6 to 10 carbon atoms, heteroaryl or a group of the formula -NR¹⁵R¹⁶,
in which
R¹⁵ and R¹⁶ are identical or different and denote hydrogen, benzyl or (C₁-C₆)-alkyl,
R¹³ and R¹⁴ are identical or different and denote hydrogen, (C₁-C₆)-alkyl or cycloalkyl having 3 to 6 carbon atoms,
R³ represents hydrogen, amino or
a radical of the formula 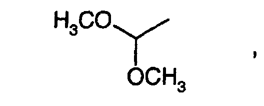 or formyl, cyano or trifluoromethyl, or
a straight-chain, branched or cyclic, saturated or unsaturated hydrocarbon radical having up to 8 carbon atoms, which is optionally mono- or polysubstituted, in an identical or different manner, by aryloxy having 6 to 10 carbon atoms, azido, cyano, hydroxyl, carboxyl, (C₁-C₆)-alkoxycarbonyl, a 5- to 7-membered heterocyclic ring, (C₁-C₆)-alkylthio, (C₁-C₆)-alkoxy, which for its part can be substituted by azido or amino,
and/or is substituted by triazolyl which for its part can be substituted up to 3 times by (C₁-C₆)-alkoxycarbonyl,
and/or can be substituted by groups of the formula -OSO₂-CH₃ or (CO)ₐ-NR¹⁷R¹⁸,
in which
a denotes a number 0 or 1,
R¹⁷ and R¹⁸ are identical or different and denote hydrogen or aryl, aralkyl having 6 to 10 carbon atoms,
or denote (C₁-C₆)-alkyl which is optionally substituted by (C₁-C₆)-alkoxycarbonyl, hydroxyl, phenyl or benzyl, where phenyl or benzyl are optionally mono- or polysubstituted, in an identical or different manner, by hydroxyl, carboxyl, (C₁-C₆)-alkyl or (C₁-C₆)-alkoxy,
or (C₁-C₆)-alkyl is optionally substituted by groups of the formulae NH-CO-CH₃ or NH-CO-CF₃,
R⁴ represents hydrogen, (C₁-C₄)-alkyl, acetyl or benzoyl,
D represents an oxygen or sulphur atom,
R⁵ represents hydrogen, halogen or straight-chain or branched alkyl having up to 6 carbon atoms,
and their salts.

2. Dihydropyrimidines according to Claim 1,
in which
R¹ represents phenyl, furyl, thienyl, pyridyl, cyclopentyl or cyclohexyl, where the abovementioned ring systems are optionally substituted one or 2 times, in an identical or different manner, by substituents selected from the group consisting of halogen, trifluoromethyl, nitro, SO₂-CF₃, methyl, cyano, trifluoromethoxy, carboxyl, methoxycarbonyl or radicals of the formula -CO-NH-CH₂-C(CH₃)₃, -CO-NH(CH₂)₂OH, -CO-NH-CH₂-C₆H₅, -CO-NH-C₆H₅, -CO-NH-(pOH)-C₆H₄, -O-CH₂-C₆H₅ or -S-pCl-C₆H₄,
R² represents a radical of the formula -OR¹² or -NR¹³R¹⁴,
in which
R¹² denotes hydrogen, (C₁-C₄)-alkenyl or (C₁-C₄)-alkyl, which is optionally substituted by pyridyl, cyano, phenoxy, benzyl or by a radical of the formula -NR¹⁵R¹⁶,
in which
R¹⁵ and R¹⁶ are identical or different and denote hydrogen, methyl or ethyl,
R¹³ and R¹⁴ are identical or different and denote hydrogen, methyl, ethyl or cyclopropyl,
R³ represents hydrogen or a radical of the formula 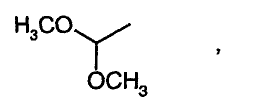 or
represents formyl, cyano, trifluoromethyl or cyclopropyl, or
represents (C₁-C₄)-alkyl which is optionally substituted by radicals of the formulae -SO₂CH₃, -NH-CO-CH₃, -NH-CO-CF₃, fluorine, chlorine, (C₁-C₃)-alkoxycarbonyl or hydroxyl,
and/or alkyl is optionally substituted by groups of the formulae -OSO₂-CH₃ or (CO)ₐ-NR¹⁷R¹⁸,
in which
a denotes a number 0 or 1,
R¹⁷ and R¹⁸ are identical or different and denote hydrogen, phenyl or benzyl, or
denote C₁-C₄-alkyl which is optionally substituted by (C₁-C₄)-alkoxycarbonyl, hydroxyl, phenyl or benzyl, where phenyl or benzyl are optionally mono- or polysubstituted, in an identical or different manner, by hydroxyl, carboxyl, (C₁-C₄)-alkyl or (C₁-C₄)-alkoxy,
and/or (C₁-C₄)-alkyl is optionally substituted by radicals of the formula -NH-CO-CH₃ or -NH-CO-CF₃,
or
R⁴ represents hydrogen, methyl, ethyl or acetyl,
D represents an oxygen or sulphur atom,
R⁵ represents hydrogen, halogen or (C₁-C₄)-alkyl,
and their salts.

3. Dihydropyrimidines according to Claim 1 or 2,
in which
R¹ represents phenyl or thienyl, where the abovementioned ring systems are optionally substituted up to 2 times, in an identical or different manner, by substituents selected from the group consisting of fluorine, chlorine, methyl, trifluoromethyl or nitro,
R² represents a radical of the formula -OR¹² or -NR¹³R¹⁴,
in which
R¹² represents hydrogen or (C₁-C₄)-alkyl,
R¹³ and R¹⁴ are identical or different and denote hydrogen or methyl,
R³ represents hydrogen or formyl, cyano, trifluoromethyl or cyclopropyl, or represents (C₁-C₃)-alkyl which is optionally substituted by fluorine, chlorine, hydroxyl, which for its part can be substituted up to 3 times by C₁-C₃-alkoxycarbonyl,
R⁴ represents hydrogen or methyl,
D represents an oxygen or sulphur atom,
R⁵ represents hydrogen, fluorine, chlorine or (C₁-C₃)-alkyl,
and their salts.

4. Dihydropyrimidines according to one of Claims 1 to 3,
in which
R¹ represents phenyl or thienyl, each of which is optionally substituted up to 2 times, in an identical or different manner, by fluorine or chlorine,
R² represents methoxy, ethoxy or n-propoxy,
R³ represents hydrogen, methyl or cyclopropyl,
R⁴ represents hydrogen,
D represents an oxygen or sulphur atom,
R⁵ represents hydrogen, fluorine or chlorine,
and their salts.

5. Process for the preparation of dihydropyrimidines of the general formula (I) or (Ia) according to one of Claims 1 to 4 in which R⁴ represents hydrogen, **characterized in that**
[A] aldehydes of the general formula (II)
R¹-CHO (II)
in which
R¹ has the meaning indicated above,
are reacted with amidines or their hydrochlorides of the formula (III) 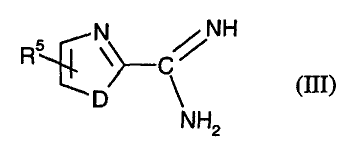 in which
R⁵ and D have the meaning indicated above,
and compounds of the general formula (IV)
R³-CO-CH₂-CO-R² (IV)
in which
R² and R³ have the meaning indicated above,
or
[B] compounds of the general formula (V) 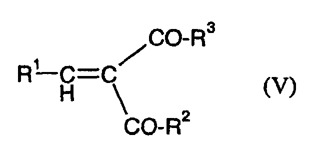 in which
R¹, R² and R³ have the meaning indicated above,
are reacted with amidines of the general formula (III) 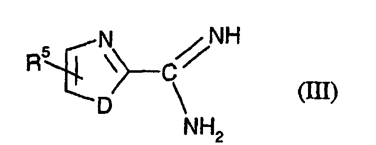 in which
R⁵ and D have the meaning indicated above,
or
[C] aldehydes of the general formula (II)
R¹-CHO (II)
in which
R¹ has the meaning indicated above,
are reacted with compounds of the general formula (VI) 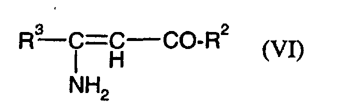 in which
R² and R³ have the meaning indicated above,
and amidines of the general formula (III).

6. Medicaments, comprising at least one compound of the general formula (I) or (Ia) according to one of Claims 1 to 4 and, if appropriate, comprising further pharmaceutical active compounds.

7. Process for the production of medicaments, **characterized in that** at least one compound of the general formula (I) or (Ia) according to one of Claims 1 to 4 is converted into a suitable administration form, if appropriate using customary auxiliaries and excipients.

8. Use of compounds of the general formula (I) or (Ia) according to one of Claims 1 to 4 for the production of a medicament.

9. Use according to Claim 8 for the production of a medicament for the treatment of acute or chronic viral diseases.

10. Use according to Claim 8 or 9 for the production of a medicament for the treatment of acute or chronic hepatitis B infections.

## Revendications

1. Dihydropyrimidines de formule générale (I) 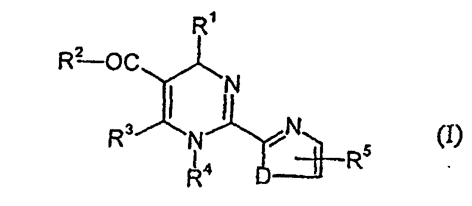 et leur forme isomère (Ia) 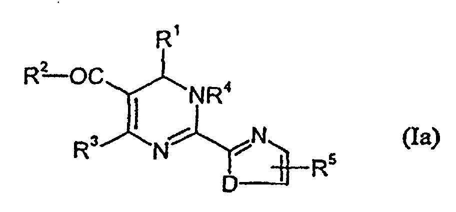 dans lesquelles
R¹ représente un groupe phényle, furyle, thiényle, pyridyle, cycloalkyle ayant 3 à 6 atomes de carbone ou un reste de formule 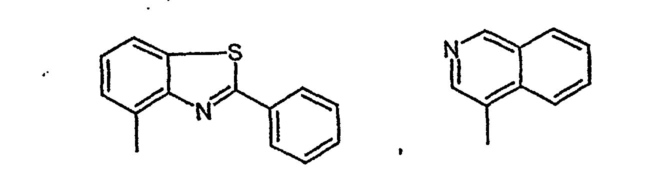 ou 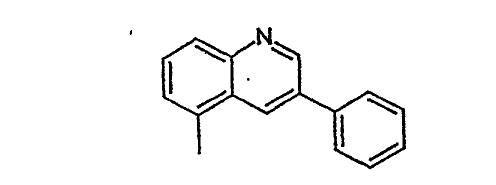 les systèmes de noyaux énumérés ci-dessus étant éventuellement substitués une ou plusieurs fois identiques ou différentes par des substituants choisis dans le groupe halogéno, trifluorométhyle, nitro, cyano, trifluorométhoxy, carboxyle, hydroxyle, alkoxy en C₁ à C₆, (alkoxy en C₁ à C₆)-carbonyle et alkyle en C₁ à C₆ qui peut lui-même être substitué par un radical aryle ayant 6 à 10 atomes de carbone ou par un halogène,
et/ou les systèmes de noyaux énumérés étant éventuellement substitués par des groupes de formules -S-R⁶, -NR⁷R⁸, CO-NR⁹R¹⁰, SO₂-CF₃ et -A-CH₂-R¹¹,
où
R⁶ désigne un groupe phényle qui est éventuellement substitué par un halogène,
R⁷, R⁸, R⁹ et R¹⁰ sont identiques ou différents et représentent l'hydrogène, un groupe phényle, phényle à substituant hydroxy, hydroxy, acyle en C₁ à C₆ ou alkyle en C₁ à C₆, qui peut être substitué de son côté par un radical hydroxy (alkoxy en C₁ à C₆)carbonyle, phényle ou phényle à substituant hydroxy,
A représente un reste O, S, SO ou SO₂,
R¹¹ désigne un groupe phényle qui est substitué le cas échéant une ou plusieurs fois identiques ou différentes par des substituants choisis dans le groupe halogéno, nitro, trifluorométhyle, alkyle en C₁ à C₆ et alkoxy en C₁ à C₆,
R² est un reste de formule -OR¹² ou -NR¹³R¹⁴,
où
R¹² représente l'hydrogène, un groupe (alkoxy en C₁ à C₆)carbonyle ou un reste hydrocarboné en C₁ à C₈ saturé ou non saturé, linéaire, ramifié ou cyclique, qui contient le cas échéant un ou deux chaînons hétéroatomiques identiques ou différents du groupe O, CO, NH, -N-(alkyle en C₁ à C₄), -N-(alkyle en C₁ à C₄)₂, S ou SO₂ et qui est substitué le cas échéant par un radical halogéno, nitro, cyano, hydroxy, aryle ayant 6 à 10 atomes de carbone ou aralkyle ayant 6 à 10 atomes de carbone, hétéroaryle ou un groupe de formule -NR¹⁵R¹⁶,
dans laquelle
R¹⁵ et R¹⁶ sont identiques ou différents et représentent l'hydrogène, un groupe benzyle ou un groupe alkyle en C₁ à C₆,
R¹³ et R¹⁴ sont identiques ou différents et représentent l'hydrogène, un groupe alkyle en C₁ à C₆ ou cycloalkyle ayant 3 à 6 atomes de carbone,
R³ représente l'hydrogène, un groupe amino ou
un reste de formule 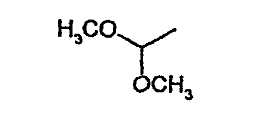 ou bien
un groupe formyle, cyano ou trifluorométhyle, ou bien
un reste hydrocarboné saturé ou non saturé linéaire, ramifié ou cyclique ayant jusqu'à 8 atomes de carbone, qui est substitué le cas échéant une ou plusieurs fois identiques ou différentes par un radical aryloxy ayant 6 à 10 atomes de carbone, azido, cyano, hydroxy, carboxyle, (alkoxy en C₁ à C₆)carbonyle, un noyau hétérocyclique pentagonal à heptagonal, un radical alkylthio en C₁ à C₆, alkoxy en C₁ à C₆, qui peut être substitué de son côté par un radical azido ou amino,
et/ou est substitué par un groupe triazolyle qui peut être substitué de son côté jusqu'à trois fois par un radical (alkoxy en C₁ à C₆)carbonyle, et/ou peut être substitué par des groupes de formules -OSO₂-CH₃ ou (CO)ₐ-NR¹⁷R¹⁸
où
a représente le nombre 0 ou 1,
R¹⁷ et R¹⁸ sont identiques ou différents et représentent l'hydrogène ou un groupe aryle, aralkyle ayant 6 à 10 atomes de carbone ou un groupe alkyle en C₁ à C₆ qui est substitué le cas échéant par un radical (alkoxy en C₁ à C₆)-carbonyle, hydroxyle, phényle ou benzyle, les radicaux phényle ou benzyle étant éventuellement substitués une ou plusieurs fois identiques ou différentes par un groupe hydroxy, carboxyle, alkyle en C₁ à C₆ ou alkoxy en C₁ à C₆,
ou bien le groupe alkyle en C₁ à C₆ est éventuellement substitué par des groupes de formules NH-CO-CH₃ ou NH-CO-CF₃,
R⁴ représente l'hydrogène, un groupe alkyle en C₁ à C₄, acétyle ou benzoyle,
D représente un atome d'oxygène ou de soufre,
R⁵ représente l'hydrogène, un halogène ou un groupe alkyle linéaire ou ramifié ayant jusqu'à 6 atomes de carbone,
et leurs sels.

2. Dihydropyrimidines suivant la revendication 1, dans lesquelles
R¹ représente un noyau phényle, furyle, thiényle, pyridyle, cyclopentyle ou cyclohexyle, les systèmes de noyaux énumérés ci-dessus étant éventuellement substitués une ou deux fois identiques ou différentes par des substituants choisis dans le groupe halogène, trifluorométhyle, nitro, SO₂-CF₃, méthyle, cyano, trifluorométhoxy, carboxyle, méthoxycarbonyle ou des restes de formule -CO-NH-CH₂-C(CH₃)₃, -CO-NH(CH₂)₂OH, -CO-NH-CH₂-C₆H₅, -CO-NH-C₆H₅, -CO-NH-(pOH)-C₆H₄, -O-CH₂-C₆H₅ ou -S-pCl-C₆H₄,
R³ représente un reste de formule -OR¹² ou -NR¹³R¹⁴,
dans laquelle
R¹² représente l'hydrogène, un groupe alcényle en C₁ à C₄ ou un groupe alkyle en C₁ à C₄ qui est substitué le cas échéant par un radical pyridyle, cyano, phénoxy, benzyle ou par un reste de formule -NR¹⁵R¹⁶,
où
R¹⁵ et R¹⁶ sont identiques ou différents et représentent l'hydrogène, un groupe méthyle ou éthyle,
R¹³ et R¹⁴ sont identiques ou différents et représentent l'hydrogène, un groupe méthyle, éthyle ou cyclopropyle,
R³ représente l'hydrogène ou un reste de formule 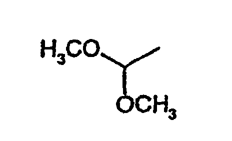 ou
représente un groupe formyle, cyano, trifluorométhyle ou cyclopropyle, ou
représente un groupe alkyle en C₁ à C₄ qui est éventuellement substitué par des restes de formules -SO₂CH₃, -NH-CO-CH₃, -NH-CO-CF₃, le fluor, le chlore, des restes de formule (alkoxy en C₁ à C₃)carbonyle ou le reste hydroxy,
et/ou le groupe alkyle est éventuellement substitué par des groupes de formules -OSO₂-CH₃ ou (CO)ₐ-NR¹⁷R¹⁸,
où
a est le nombre 0 ou 1,
R¹⁷ et R¹⁸ sont identiques ou différents et représentent l'hydrogène, un groupe phényle ou benzyle, ou bien
représentent un groupe alkyle en C₁ à C₄ qui est éventuellement substitué par un radical (alkoxy en C₁ à C₄)carbonyle, hydroxyle, phényle ou benzyle, les radicaux phényle ou benzyle étant éventuellement substitués une ou plusieurs fois identiques ou différentes par un groupe hydroxy, carboxy, alkyle en C₁ à C₄ ou alkoxy en C₁ à C₄, et/ou le groupe alkyle en C₁ à C₄ est éventuellement substitué par des restes de formules -NH-CO-CH₃ ou -NH-CO-CF₃,
ou bien
R⁴ représente l'hydrogène, un groupe méthyle, éthyle ou acétyle,
D est un atome d'oxygène ou de soufre,
R⁵ représente l'hydrogène, un halogène ou un groupe alkyle en C₁ à C₄,
et leurs sels.

3. Dihydropyrimidines suivant la revendication 1 ou 2,
dans lesquelles
R¹ est un noyau phényle ou thiényle, les noyaux indiqués ci-dessus étant éventuellement substitués jusqu'à deux fois identiques ou différentes par des substituants choisis dans le groupe fluor, chlore, méthyle, tifluorométhyle ou nitro,
R² représente un reste de formule -OR¹² ou -NR¹³R¹⁴,
dans laquelle
R¹² représente l'hydrogène ou un groupe alkyle en C₁ à C₄
R¹³et R¹⁴ sont identiques ou différents et représentent l'hydrogène ou le groupe méthyle,
R³ représente l'hydrogène ou un groupe formyle, cyano, trifluorométhyle ou cyclopropyle,
ou représente un groupe alkyle en C₁ à C₃, qui est éventuellement substitué par du fluor, du chlore ou le radical hydroxy, qui peut être substitué à son tour jusqu'à trois fois par un radical (alkoxy en C₁ à C₃)-carbonyle,
R⁴ représente l'hydrogène ou le groupe méthyle,
D représente un atome d'oxygène ou de soufre,
R⁵ représente l'hydrogène, le fluor, le chlore ou un groupe alkyle en C₁ à C₃,
et leurs sels.

4. Dihydropyrimidines suivant l'une des revendications 1 à 3,
dans lesquelles
R¹ est un noyau phényle ou thiényle qui est éventuellement substitué jusqu'à deux fois identiques ou différentes par du fluor ou du chlore,
R² est un groupe méthoxy, éthoxy ou n-propoxy,
R³ représente l'hydrogène, un groupe méthyle ou cyclopropyle,
R⁴ représente l'hydrogène,
D représente un atome d'oxygène ou de soufre,
R⁵ représente l'hydrogène, le fluor ou le chlore,
et leurs sels.

5. Procédé de production de dihydropyrimidines de formule générale (I) ou (Ia) suivant l'une des revendications 1 à 4, dans lesquelles R⁴ représente l'hydrogène, **caractérisé en ce qu'**on fait réagir
[A] des aldéhydes de formule générale (II)
R¹-CHO (II)
dans laquelle
R¹ a la définition indiquée ci-dessus,
avec des amidines ou leurs chlorhydrates de formule (III) 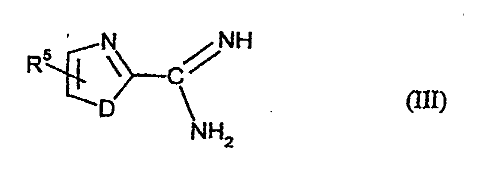 dans laquelle
R⁵ et D ont la définition indiquée ci-dessus,
et des composés de formule générale (IV)
R³-CO-CH₂-CO-R² (IV)
dans laquelle
R² et R³ ont la définition indiquée ci-dessus,
ou bien
[B] des composés de formule générale (V) 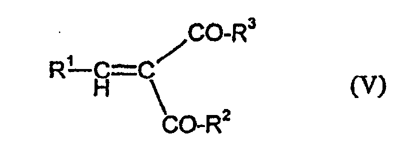 dans laquelle
R¹, R² et R³ ont la définition indiquée ci-dessus, avec des amidines de formule générale (III) 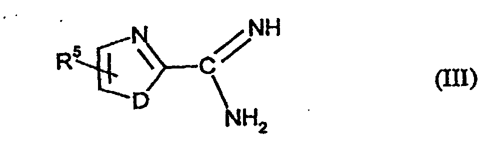 dans laquelle
R⁵ et D ont la définition indiquée ci-dessus,
ou bien
[C] des aldéhydes de formule générale (II)
R¹-CHO (II)
dans laquelle
R¹ a la définition indiquée ci-dessus,
avec des composés de formule générale (VI) 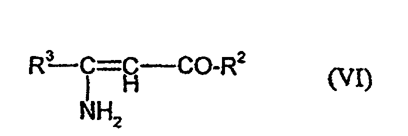 dans laquelle
R² et R³ ont la définition indiquée ci-dessus,
et des amidines de formule générale (III).

6. Médicament contenant au moins un composé de formule générale (I) ou (Ia) suivant l'une des revendications 1 à 4 et contenant éventuellement d'autres substances pharmaceutiques actives.

7. Procédé de préparation de médicaments, **caractérisé en ce qu'**on fait prendre une forme d'administration appropriée à au moins un composé de formule générale (I) ou (Ia) suivant l'une des revendications 1 à 4, en utilisant éventuellement des substances auxiliaires et des supports classiques.

8. Utilisation de composés de formule générale (I) ou (Ia) suivant l'une des revendications 1 à 4 pour la préparation d'un médicament.

9. Utilisation suivant la revendication 8 pour la préparation d'un médicament destiné au traitement de maladies virales aiguës ou chroniques.

10. Utilisation suivant la revendication 8 ou 9, pour la préparation d'un médicament destiné au traitement d'affections aiguës ou chroniques du type de l'hépatite B.
